# EUROPEAN PATENT SPECIFICATION

(11) **EP 3 544 594 B1**
(45) Date of publication and mention of the grant of the patent: **25.08.2021**
(21) Application number: 17808050.3
(22) Date of filing: 24.11.2017
(51) Int. Cl.: A61K 9/51, A61K 47/69, A01N 25/28, A01N 31/16, A61K 31/085

(54) **MESOPOROUS SILICA MATERIALS FOR THE CONTROLLED RELEASE OF ACTIVE SUBSTANCES AND THEIR APPLICATIONS**
MESOPORÖSE KIESELSÄUREMATERIALIEN ZUR KONTROLLIERTEN FREISETZUNG VON WIRKSTOFFEN UND DEREN ANWENDUNGEN
MATÉRIAUX DE SILICE MÉSOPOREUSE POUR LA LIBÉRATION CONTRÔLÉE DE SUBSTANCES ACTIVES ET LEURS APPLICATIONS

(30) Priority: 25.11.2016 CZ 20160735
(43) Date of publication of application: 02.10.2019
(73) Proprietor: Universitat Politècnica de València, 46022 Valencia (ES); Czech University of Life Sciences Prague, 165 00 Prague (CZ)
(72) Inventor: BERNARDOS BAU, Andrea, 46022 Valencia (ES); MARTINEZ MAÑEZ, Ramón, 46022 Valencia (ES); KLOUCEK, Pavel, 165 00 Prague (CZ); BOZIK, Matej, 165 00 Prague (CZ)
(74) Representative: Pons Ariño, Angel
(86) International application number: PCT/EP2017/080341
(87) International publication number: WO 2018/096098

(56) References cited:
- WO-A1-2015/044673
- ANDREA BERNARDOS ET AL: "Enzyme-Responsive Intracellular Controlled Release Using Nanometric Silica Mesoporous Supports Capped with "Saccharides"", ACS NANO, vol. 4, no. 11, 23 November 2010 (2010-11-23), pages 6353-6368, XP55233896, US ISSN: 1936-0851, DOI: 10.1021/nn101499d
- ANEZKA JANATOVA ET AL: "Long-term antifungal activity of volatile essential oil components released from mesoporous silica materials", INDUSTRIAL CROPS AND PRODUCTS., vol. 67, 1 May 2015 (2015-05-01), pages 216-220, XP55443108, NL ISSN: 0926-6690, DOI: 10.1016/j.indcrop.2015.01.019 cited in the application

## Description

### Field of the invention

The present invention relates to the field of porous materials based on silicon dioxide for the controlled release of active substances and their uses.

### Background of the invention

In recent decades, develop of antimicrobial formulations has given rise to new concepts related to the design of devices allowing bioactive compounds release such essential oil components. In most cases, traditional delivery systems are based on the use of organic polymers, which usually release their cargo via diffusion-controlled processes or through the degradation of the polymeric matrix. Moreover, controlled release of certain substances when the release happens only in the presence of specific stimulus becomes to be an innovative idea.

Instead traditional delivery systems, mesoporous silica support (SMPS) functionalized with 'molecular gates' has constituted a promising research field last years. The use of SMPS with the pores capped by 'molecular gates' that could be opened with different stimuli would guarantee the complete arrival of the cargo to the action point. Different external triggers (photochemical, electrochemical, ionic, polarity changes, presence of certain bio-molecules, etc.) have been employed until the present-day to release a wide variety of guest molecules (cytotoxic agents, proteins, DNA and RNA fragments, enzymes, etc.).

*Aspergillus niger* (A. niger) is an important pathogen that is stored in agricultural products, some of its strains produce ochratoxin A, with nephrotoxic, immunotoxic, carcinogenic and teratogenic effects. On the other hand, volatile antimicrobials from plants are an adequate alternative to synthetic pesticides and food preservatives, and their controlled release can facilitate their application and provide long-term effectiveness and easy handling, without affecting their fungicidal activity.

The encapsulation of volatile antimicrobial in particular systems such as MCM-41-type has been previously studied in A. Janatova et al. Industrial Crops and Products, 2015, 67, 2016-2020. The adsorption of the volatile compounds into the mesopores of the MCM-41 type controlled the evaporation of the volatile compounds, and therefore increase their antifungal effect against *Aspergillus niger.*

WO 2015/044673 discloses a delivery system for a biocide comprising a core material loaded with a biocide, and a capping material applied to the core material so as to contain the biocide, and enable exposure of the biocide when the capping material is broken down or opened up. However, some essential oils encapsulated into the materials mentioned above did not release the volatile active principle only in the presence of pathogenic microorganisms, and therefore, these materials were not able to reduce the evaporation of the active principle and to release them in a controlled manner.

### Summary of the invention

The present invention relates to mesoporous silica nanoparticles for the controlled release of active substances against a characteristic microorganism, where the silica nanoparticles are characterized by:
- a size comprised between 100 nm to 1 µm (micron) and a pore size between 2 to 15 nm, and on the surface of these materials, sugar derivatives are functionalized by the use of alkoxysilane molecules that act as an anchor molecule for the formation of the covalent bond with the surface of the material; and the carbohydrate derivatives used contain saccharides from 3 to 20 units, and are functionalized on the surface of nanoparticles encapsulated with active substance;
- the active substance is a volatile antibacterial and / or antifungal substance; and
- the carbohydrate derivative is maltodextrin comprising 3 to 20 units of monosaccharides.

The present patent describes a new antimicrobial formulation using SMPS loaded with essential oil components and functionalized with 'sugar gates' derivatives. In this invention, the presence of a unique microorganisms, the fungi *Aspergillus niger,* produces exogenous amylases required for hydrolysis of sugar and allows the essential oil components release from the pores. The invention is in the field of design a new opening-closing mechanism based on the use of exogenous enzymes from the fungi *Aspergillus niger.* The application of enzymes as stimuli on the gate-like functional hybrid systems improves natural bioactive components controlled release. Using this approach, the active compounds remains encapsulated into the mesoporous material until the presence of the target microorganisms. Moreover, the unnecessary and premature release of the active substance into the environment is eliminated. In addition, this process allows the long-term application of volatile antimicrobial substances, which would otherwise quickly evaporate.

The summary of the invention resides in a process for developing new materials for the controlled release of active substances. This material comprises a mesoporous silica support coated in oligosaccharides. The active substances are entrapped inside the pores. The degradation of the molecules covalently bound to the porous silica, is produced by the enzymatic activity, which can be produced by the microorganisms present in the environment of the material (the target microorganism). This microorganism is capable of hydrolysing the oligosaccharides which coat the support, thus releasing the load. Since the active ingredient loaded in the pores has antimicrobial properties, it will inhibit the growth of microorganisms present in the environment of the material.

The molecules coated the SMPS are covalently bound to the surface of the mesoporous nanoparticles, by binding to alkoxysilanes, which form covalent bonds with the silanol groups on the surface of the nanoparticles. The antimicrobial agents acting such as active substances are essential oils or components thereof, for example, carvacrol, eugenol, thymol, thymoquinone, diallyl disulfide or allyl isothiocyanate. The mesoporous silica nanoparticles for the controlled release of volatile active substances have a great potential for applications in the food, agricultural, cosmetic and pharmaceutical industries, due to their great stability and bioavailability.

The present invention is based on a mesoporous silica material, for the controlled release of active substances in the presence of a certain stimulus such as a microorganism. The mesoporous silica nanomaterial comprises particle size in the range between 100 nm to 1 micron, with a pore size of 2 to 15 nm. These materials have a specific surface capable of forming covalent bonds with carbohydrate derivatives with a number of saccharide units from 1 to 20, through trialkoxysilane molecules that act as anchoring molecules. These bonds are produced outside the pores of nanoparticles with active substances loaded. The anchoring molecules are preferably selected from the group consisting of 3-aminopropyltrietioxysilane, 3-iodopropyltriethoxysilane and 3-mercaptopropyltrimethoxysilane. The mesoporous material of the present invention is preferably under vigorous agitation of the active substance and the mesoporous material without solvent, at a temperature comprises from 20 ° C to 250 ° C, preferably during at least 24 hours. The sugar derivative is then added to the reaction mixture, previously derivatized with the alkoxy-3-aminopropyltriethoxysilane. This derivatization is carried out with a suspension of a saccharide (1 to 20 saccharide units) in ethanol, at a temperature ranging from 20 ° C to 80 ° C, preferably for at least 24 hours. The resulting mixture of the nanoparticles loaded with active substance and the sugar derivative is stirred at a temperature compromises from 20 ° C to 80 ° C, preferably for 4-8 hours.

In another preferred embodiment of the first aspect of the present invention, the active substance encapsulated in the mesoporous material, the volatile antibacterial and / or antifungal, is selected from the group consisting of eugenol, carvacrol, cinnamaldehyde, thymol, allyl isothiocyanate, diallyl disulfide, thymoquinone, citral, linalool, geraniol, menthol and mixtures thereof.

In another preferred embodiment of the first aspect of the present invention, the mesoporous material of the present invention is based on mesoporous silica nanoparticles selected from the group consisting of MCM-41 (spherical particles with a diameter of 100 to 1000 nm and 2-3 nm pore diameter) and SBA-15 (length of about 1000 nm, and a pore diameter of 5 to 15 nm).

In another preferred embodiment of the first aspect of the present invention, the saccharide derivative is maltodextrin with a number of saccharide comprised between 3-20 units.

The present invention also provides the use of mesoporous materials as antimicrobial agents, preferably antifungal agents.

The present invention also provides the use of mesoporous material of the present invention in the agricultural and / or food industries and / or in the cosmetic industry.

The present invention is also based on the mesoporous material of the present invention for use as a medicament.

### Brief description of the figures

Figure 1: Schematic representation of mesoporous silica material encapsulated with EOCs (in this figure eugenol) and capped with maltodextrin and maltose.
Figure 2: Schematic representation of the synthesis of hybrid mesoporous nanoparticles material.
Figure 3: Schematic representation of the release of eugenol encapsulated in a mesoporous silica material covalently bound to maltodextrins, by the enzymatic hydrolysis of the o-glycosidic bonds that form the maltodextrin.
Figure 4: Kinetic release profile of eugenol from Maltodextrin-SMPS-EU in the absence and in the presence of fungi *A*. *Niger.*
Figure 5: The antifungal activity of eugenol encapsulated in silica and capped with sugar derivative: Glucose-SMPS-EU, Maltose-SMPS-E and, Maltodextrin-SMPS-EU after 15 days.
Figure 6: Amination reaction between alkoxysilanes and sugars to obtain the corresponding glucose, maltose and maltodextrin derivatives.

### Examples

### Example 1: Synthesis of MCM-41

The MCM-41 mesoporous nanoparticles were synthesized using the following procedure: n-cetyltrimethylammoniumbromide (CTABr, 2.00 g, 5.48 mmol) was first dissolved in 960 mL of deionized water. NaOH (aq) (2.00 M, 7.00 mL) was added to the CTABr solution, followed by adjusting the solution temperature to 95°C. TEOS (10.00 mL, 5.14.10-2 mol) was then added dropwise to the surfactant solution. The mixture was allowed to stir for 3 h to give a white precipitate. The solid product was centrifuged, and washed with deionized water and ethanol. Finally the solid was dried at 60°C (MCM-41 as-synthesized). To prepare the final porous material (MCM-41), the as-synthesized solid was calcined at 550 °C using an oxidant atmosphere for 5 h in order to remove the template phase. This material will be called SMPS (mesoporous solid support).

### Example 2: Synthesis of sugar derivatives

### Synthesis of the Glucose derivative (not according to the invention)

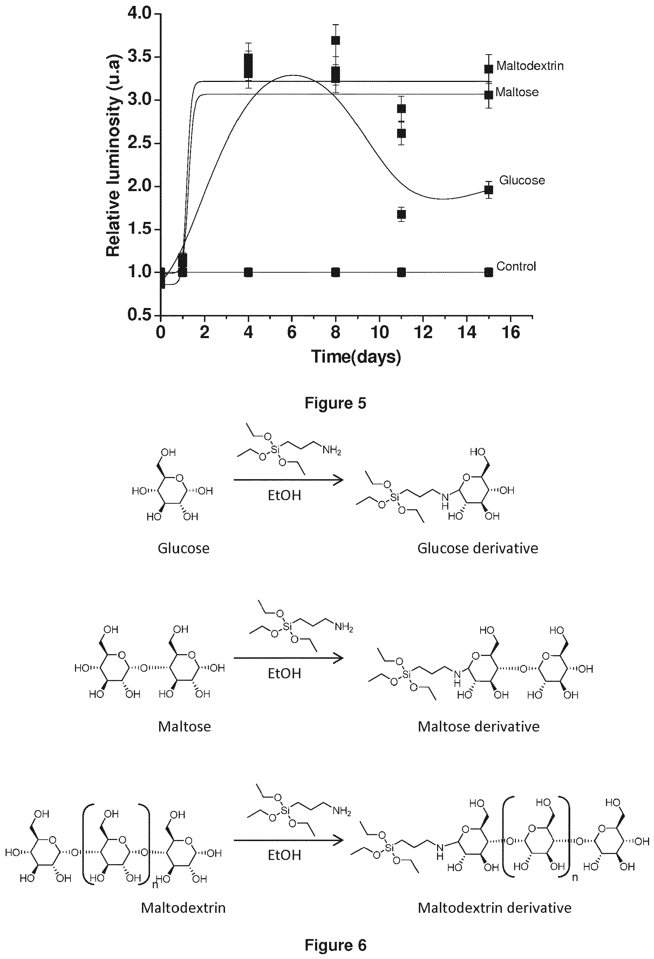

A solution of 3-aminopropyltriethoxysilane (5.85 mL, 25 mmol) in ethanol was added to a suspension of glucose (5.4 g, 15 mmol), in ethanol (total volume 250 mL). The reaction mixture was stirred for 24 h at room temperature and then heated at 60 °C for 30 min. The solvent was evaporated under reduced pressure to give a white solid, GLU (see Figure 6).

### Synthesis of the Maltose derivative (not according to the invention)

A solution of 3-aminopropyltriethoxysilane (5.85 mL, 25 mmol) in ethanol was added to a suspension of maltose monohydrate (5.4 g, 15 mmol), in ethanol (total volume 250 mL). The reaction mixture was stirred for 24 h at room temperature and then heated at 60 °C for 30 min. The solvent was evaporated under reduced pressure to give a white solid, MAL (see Figure 6).

### Synthesis of the Maltodextrin derivative

A solution of 3-aminopropyltriethoxysilane (5.85 mL, 25 mmol) in ethanol was added to a suspension of maltodextrin 18% (5.4 g), in ethanol (total volume 250 mL). The reaction mixture was stirred for 24 h at room temperature and then heated at 60 °C for 30 min. The solvent was evaporated under reduced pressure to give a white solid, MDX (see Figure 6).

### Example 3: Synthesis of SMPS-EU

Silica loading with eugenol was achieved via vapor adsorption by mixing each eugenol (50 mg) with the MCM-41 silica solid (50 mg) in a tightly closed vial. The mixture was incubated in an oven at 40 °C for 24 h while being continuously shaken. The amount of eugenol loaded in the MCM-41 support was determined by monitoring the sample weight increase and by Thermogravimetric analysis TGA and elemental analysis (EA) studies. Using this approach, the final mesoporous materials SMPS-EU was prepared, with a 51.6% of organic content.

### Example 4: Sugar derivatives functionalization into mesoporous materials

### Synthesis of GLU-SMPS-EU (not according to the invention)

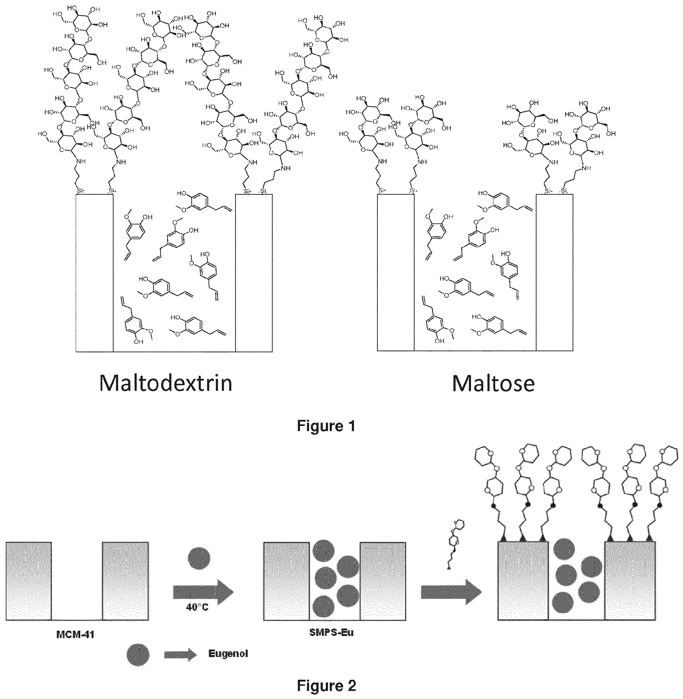

In a typical synthesis, 100 mg of SMPS-EU were suspended in 40 mL of water in a round-bottomed flask under inert atmosphere. Then, an excess of the GLU (1g, 1.8 mmol) in 20 mL of water was added, and the final mixture was stirred for 5.5 h at room temperature. Finally, the solid (GLU-SMPS-EU) was filtered off, washed with 40 mL of water, and dried at 40 °C for 12 h (see Figure 2). The yield of the reaction was 40%, and the eugenol content was 10% by weight.

### Synthesis of MAL-SMPS-EU (not according to the invention)

In a typical synthesis, 100 mg of SMPS-EU were suspended in 40 mL of water in a round-bottomed flask under inert atmosphere. Then, an excess of MAL (1g, 1.8 mmol) in 20 mL of water was added, and the final mixture was stirred for 5.5 h at room temperature. Finally, the solid (MAL-SMPS-EU) was filtered off, washed with 40 mL of water, and dried at 40 °C for 12 h (see Figure 2). The yield of the reaction was 40%, and the eugenol content was 10% by weight.

### Synthesis of MDX-SMPS-EU

In a typical synthesis, 100 mg of SMPS-EU were suspended in 40 mL of water in a round-bottomed flask under inert atmosphere. Then, an excess of MDX (1g) in 20 mL of water was added, and the final mixture was stirred for 5.5 h at room temperature. Finally, the solid (MDX-SMPS-EU) was filtered off, washed with 40 mL of water, and dried at 40 °C for 12 h. The yield of the reaction was 40%, and the eugenol content was 10% by weight.

### Example 5: Eugenol release studies

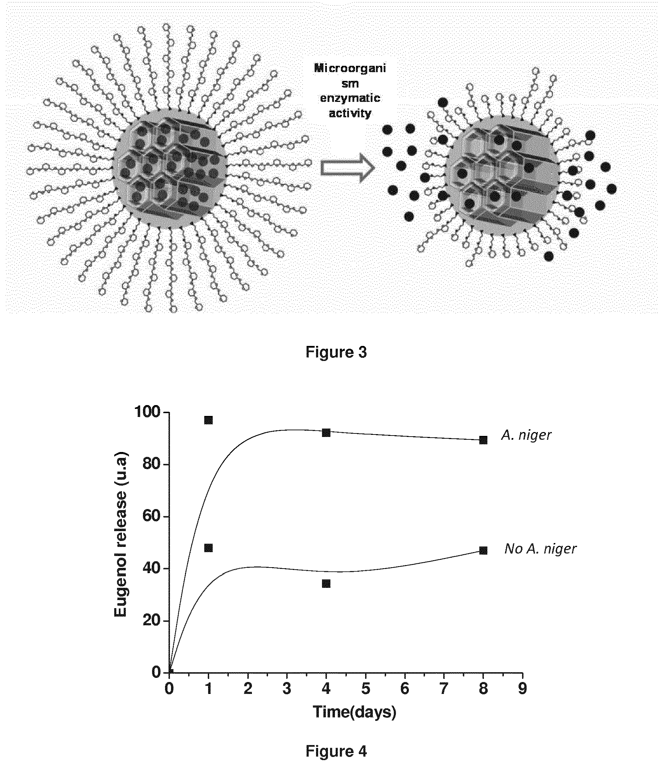

In a typical experiment, 5 mg of MDX-SMPS-EU are suspended in 100 µL of sterilized water and are placed into vials contained Sabouraud Dextrose agar (SDA). Spore suspension was prepared by washing the *Aspergillus niger* culture with 2 mL sterile Tris Buffered Saline (TBS) containing 1% of Tween 80 and standardized to 1 McFarland (approx. 5×10⁶ spores per mL). The 300 µL of sterile TBS and 50 µL of the spore suspension were added to the vials containing MDX-SMPS-EU. Next, growth control, MDX-SMPS-EU with fungi and MDX-SMPS-EU without fungi were tested in triplicate. Static headspace gas chromatography (GC) is the technique used for the analysis of volatile eugenol. The presence of exogenous enzymes from *A*. *niger* is able to hydrolyze the glycosidic bonds that form the structure of the sugar that acts as a gate blocking the exit of the pores. This enzyme allows the release of volatile eugenol from the porous support, see Figure 3. At a certain time solid-phase microextraction (SPME) is using to get eugenol evaporated from the vials. The delivery of eugenol from the pore voids to the agar is monitored via the vapor release using static headspace and GC.

### Results:

As stated above, the aim is to develop enzyme-fungi-triggered delivery systems using saccharide-capped mesoporous nanoscopic scaffolds loaded with eugenol. *Aspergillus niger* uncap the pores through selective hydrolysis of the 1-4 glycosidic bonds in the maltodextrin. In a typical experiment, MDX-SPMS-EU is placed in agar in the presence of and in the absence of the fungi. As a significant feature, the 'less-release' obtained in the absence of fungi should be noted. In clear contrast, the solids displayed an enzyme-dependent cargo delivery. In more detail, solid MDX-SMPS-EU releases after 24 h in the presence of fungi MDX-SMPS-EU almost 60% of the cargo more than solid in absence of the fungi (see Figure 4).

### Example 6: In vitro Antifungal test.

Spore suspension was prepared by washing the *Aspergillus niger* culture with 2 mL sterile Tris Buffered Saline (TBS) containing 1% of Tween 80 and standardized to 1 McFarland (approx. 5×10⁶ spores per mL). The 300 µL of sterile TBS and 50 µL of the spore suspension were added to the Eppendorf tubes containing the different EOCs formulations that differ in the length of the carbohydrate chains that act as molecular gates (GLU-SMPS-EU, MAL-SPMS-EU and MDX-SMPS-EU). In addition, a control with pure eugenol and eugenol encapsulated in MCM-41 without molecular gate (SMPS-EU) are used. All the mixtures were then vigorously vortexed to ensure complete suspension and immediately spread onto Petri dishes which contained Sabouraud Dextrose agar (SDA). The petri dishes were not sealed in order to allow the vapors to escape. Next, growth control (without eugenol), pure eugenol, SMPS-EU, GLU-SMPS-EU, MAL-SPMS-EU and MDX-SMPS-EU are tested in triplicate. These tests are realized with similar amount (0.5 mg) of eugenol: i.e. 0.5 mg of pure, 1 mg of SPMS-EU and 5 mg of Sugar-SPMS-EU. Monitoring was done for two weeks by visual inspection. This relative luminosity was close to 0 the greater the growth of the fungus, and close to 4 a.u. when greater was the antifungal activity, see Figure 5.

### Results:

The initial effect of the eugenol on the growth of *Aspergillus niger* was not obvious, hence after 2 days all there are no significant differences in comparison to the control. After 7 days of exposure, the most active formulation was MAL-SMPS-EU and MDX-SMPS-EU. After two weeks, only MAL-SPMS-EU and MDX-SPMS-EU systems maintain antifungal activity and inhibited fungal growth.

### Example 7: Synthesis of mesoporous materials functionalized with sugars and encapsulated with active substances

According to Examples 3 and 4 under the same reaction conditions, the mesoporous materials listed in Table 1 have been prepared. Only the materials comprising maltodextrin (MDX) are according to the invention.

**Table 1:**

| Mesoporous Materials | Active compound | Nanoparticles SiO₂ | Sugar derivative | Active compound content (weight%) | Yield (%) |
|---|---|---|---|---|---|
| MAL-SMPS-CAR | carvacrol | MCM-41 | MAL | 10 | 40 |
| MDX-SMPS-CAR | carvacrol | MCM-41 | MDX | 10 | 40 |
| MDX-SMPS-CIN | cinnamaldehyde | MCM-41 | MAL | 10 | 40 |
| MDX-SMPS-CIN | cinnamaldehyde | MCM-41 | MDX | 10 | 40 |
| MAL-SMPS-THY | thymol | MCM-41 | MAL | 10 | 40 |
| MDX-SMPS-THY | thymol | MCM-41 | MDX | 10 | 40 |
| MAL-SMPS-AITC | allyl isothiocyanate | MCM-41 | MAL | 10 | 40 |
| MDX-SMPS-AITC | allyl isothiocyanate | MCM-41 | MDX | 10 | 40 |
| MAL-SMPS-DAD | diallyl disulfide | MCM-41 | MAL | 10 | 40 |
| MDX-SMPS-DAD | diallyl disulfide | MCM-41 | MDX | 10 | 40 |
| MAL-SMPS-TQ | thymoquinone | MCM-41 | MAL | 10 | 40 |
| MDX-SMPS-TQ | thymoquinone | MCM-41 | MDX | 10 | 40 |
| MAL-SMPS-CIT | citral | MCM-41 | MAL | 10 | 40 |
| MDX-SMPS-CIT | citral | MCM-41 | MDX | 10 | 40 |
| MAL-SMPS-LIN | linalool | MCM-41 | MAL | 10 | 40 |
| MDX-SMPS-LIN | linalool | MCM-41 | MDX | 10 | 40 |
| MAL-SMPS-GER | geraniol | MCM-41 | MAL | 10 | 40 |
| MDX-SMPS-GER | geraniol | MCM-41 | MDX | 10 | 40 |
| MAL-SMPS-MENT | menthol | MCM-41 | MAL | 10 | 40 |
| MDX-SMPS-MENT | menthol | MCM-41 | MDX | 10 | 40 |

### Industrial applicability

The technical solution relates to an active substance formulation for non-toxic inert carriers, allowing the release in a controlled point and time necessary for its application. The release is controlled by the enzymatic activity at the target site, for example, at the site of a pathogenic microorganism. This solution can be used advantageously for the application of substances, which in pure state have suitable technological properties but which are volatile, have undue polarity or other unsuitable physical-chemical properties.

## Claims

1. Mesoporous silica nanoparticles for the controlled release of active substances against a characteristic microorganism, where the silica nanoparticles are **characterized by**:
- a size comprised between 100 nm to 1 µm and a pore size between 2 to 15 nm, and on the surface of these materials, sugar derivatives are functionalized by the use of alkoxysilane molecules that act as an anchor molecule for the formation of the covalent bond with the surface of the material; and the carbohydrate derivatives used contain saccharides from 3 to 20 units, and are functionalized on the surface of nanoparticles encapsulated with active substance;
- the active substance is a volatile antibacterial and / or antifungal substance; and
- the carbohydrate derivative is maltodextrin comprising 3 to 20 units of monosaccharides.

2. Mesoporous silica nanoparticles according to claim 1, **characterized by** the selected mesoporous silica nanoparticles consist of MCM-41 type and SBA-15.

3. Mesoporous silica nanoparticles according to any of claims 1 or 2, wherein the alkoxysilane molecules are 3-aminopropyl triethoxy silane.

4. The use of mesoporous silica nanoparticles according to any of claims 1 to 3, in food and / or agriculture industries and / or in the cosmetics industry.

5. Mesoporous material according to any of claims 1 to 3 for use as a medicine.

## Patentansprüche

1. Mesoporöse Siliziumdioxid-Nanoteilchen zur kontrollierten Freisetzung von Wirkstoffen gegen einen charakteristischen Mikroorganismus, wobei die Siliziumdioxid-Nanoteilchen **gekennzeichnet sind durch**:
- eine Größe zwischen 100 nm bis 1 µm und eine Porengröße zwischen 2 bis 15 nm, wobei auf der Oberfläche dieser Materialien Zuckerderivate durch die Verwendung von Alkoxysilanmolekülen funktionalisiert werden, die als Ankermolekül für die Bildung der kovalenten Bindung mit der Oberfläche des Materials fungieren; und die verwendeten Kohlenhydratderivate Saccharide von 3 bis 20 Einheiten enthalten und auf der Oberfläche von wirkstoffverkapselten Nanoteilchen funktionalisiert sind;
- der Wirkstoff ein flüchtiger antibakterieller und / oder antimykotischer Stoff ist; und
- das Kohlenhydratderivat Maltodextrin ist, das 3 bis 20 Einheiten Monosaccharide umfasst.

2. Mesoporöse Siliciumdioxid-Nanoteilchen nach Anspruch 1, **gekennzeichnet dadurch, dass** die ausgewählten mesoporösen Siliziumdioxid-Nanoteilchen aus MCM-41-Typ und SBA-15 bestehen.

3. Mesoporöse Siliciumdioxid-Nanoteilchen nach einem der Ansprüche 1 oder 2, wobei die Alkoxysilanmoleküle 3-Aminopropyltriethoxysilan sind.

4. Verwendung von mesoporösen Siliziumdioxid-Nanoteilchen nach einem der Ansprüche 1 bis 3 in der Lebensmittel- und/oder Landwirtschaftsindustrie und / oder in der Kosmetikindustrie.

5. Mesoporöses Material nach einem der Ansprüche 1 bis 3 zur Verwendung als ein Medikament.

## Revendications

1. Nanoparticules de silice mésoporeuse pour la libération contrôlée de substances actives contre un micro-organisme particulier, où les nanoparticules de silice sont **caractérisées par** :
- une taille comprise entre 100 nm et 1 µm et une taille de pores comprise entre 2 et 15 nm, et sur la surface de ces matériaux, des dérivés de sucre sont fonctionnalisés par l'utilisation de molécules d'alcoxysilane qui agissent comme une molécule d'ancrage pour la formation de la liaison covalente avec la surface du matériau ; et les dérivés glucidiques utilisés contiennent des saccharides de 3 à 20 unités, et sont fonctionnalisés sur la surface de nanoparticules encapsulées avec la substance active ;
- la substance active est une substance volatile antibactérienne et/ou antifongique ; et
- le dérivé glucidique est la maltodextrine comprenant 3 à 20 unités de monosaccharides.

2. Nanoparticules de silice mésoporeuse selon la revendication 1, **caractérisées par le fait que** les nanoparticules de silice mésoporeuse sélectionnées sont de type MCM-41 et SBA-15.

3. Nanoparticules de silice mésoporeuse selon l'une quelconque des revendications 1 ou 2, dans lesquelles les molécules d'alcoxysilane sont du 3-aminopropyl triéthoxy silane.

4. Utilisation de nanoparticules de silice mésoporeuse selon l'une quelconque des revendications 1 à 3, dans les industries alimentaires et/ou agricoles et/ou dans l'industrie cosmétique.

5. Matériau mésoporeux selon l'une quelconque des revendications 1 à 3 pouvant être utilisé comme médicament.
